# DEMANDE DE BREVET EUROPEEN

(11) **EP 1 279 413 A1**
(43) Date de publication de la demande: **29.01.2003**
(21) Numéro de dépôt: 02356148.3
(22) Date de dépôt: 26.07.2002
(51) Int. Cl.: A61N 1/05

(54) **Sonde de rééducation périno-sphinctérienne à déformation élastique**

(30) Priorité: 27.07.2001 FR 0110165
(71) Demandeur: Electroformage Medical Concept Thiers, 63300 Thiers (FR)
(72) Inventeur: Bonnefoy, Hervé, 63920 Peschadoires (FR)
(74) Mandataire: Schouller, Jean-Philippe

(57) **Abrégé**

Cette sonde comprend un corps (1) globalement allongé et élastiquement déformable (5) qui est principalement formé à partir d'un élément filiforme (3) recourbé sur lui-même de manière à articuler l'un à l'autre ses deux brins (6, 7). Cette sonde est équipée d'au moins une électrode (2, 2'). L'élément filiforme (3) est au moins partiellement noyé à l'intérieur d'une masse de matière souple (4) qui se prolonge, à son extrémité distale (9) sous la forme d'un organe (10, 10') de mise en butée extérieure du corps (1) de la sonde contre le patient.

## Description

L'invention est du domaine de l'électrothérapie, et plus particulièrement de la rééducation périnéo-sphinctérienne. Elle a pour objet une sonde endocavitaire pour la rééducation par stimulation électrique, des muscles environnent les cavités vaginale ou anale notamment.

On connaît dans le domaine de l'électrothérapie des sondes endocavitaires prévues pour être introduites dans une cavité du patient, vaginale ou anale notamment, en vue d'y appliquer des stimulations électriques. Ces sondes permettent souvent de communiquer également des informations à un centre de calcul sur les effets produits par ces stimulations.

Un premier problème posé par cette technique réside dans le positionnement et l'immobilisation de la sonde à l'intérieur de la cavité.

Selon une première technique connue, ces sondes comprennent une électrode unique filiforme, qui est repliée en boucle pour être introduite dans la cavité du patient, et qui est maintenue en conformation par un support qui prend un appui extérieur contre le patient. Ce support comporte en outre les moyens de raccordement de l'électrode à une source extérieure de tension. On pourra notamment se reporter aux brevets US6246915 (BOUTOS), US6151527 (BOUTOS), US5782902 (BOUTOS) qui décrivent de telles sondes.

Il apparaît que l'appui extérieur que prend l'électrode par l'intermédiaire de son support ne garantit pas son positionnement idoine et son immobilisation à l'intérieur du muscle. Par ailleurs, la souplesse de l'électrode ne garanti pas non plus son application correcte contre la paroi interne du muscle, notamment en des endroits déterminés essentiels pour l'obtention d'un traitement efficace.

Un autre problème posé par cette technique, relativement ancienne, réside dans le fait que de la paroi intérieure du muscle est stimulée sur la totalité de sa longueur. Or, les stimulations appliquées par les sondes visent à résoudre un problème du patient déterminé, tel que l'incontinence. Il en découle qu'il est inutile de stimuler en totalité les muqueuses du patient, sensibles, alors que seules certaines zones du muscle suffisent à être stimulées pour l'obtention d'un traitement satisfaisant.

C'est pourquoi il a été proposé des sondes d'un deuxième type, qui comprennent principalement un corps allongé globalement cylindrique et rigide, agencé pour être logé à l'intérieur du vagin. Ce corps supporte à sa périphérie au moins une électrode, qui est reliée à une source extérieure de tension pour appliquer la stimulation, et le cas échéant qui est reliée au centre de calcul pour envoyer des informations vers celui-ci. Les électrodes sont placées en des endroits déterminés du corps de la sonde, qui correspondent aux zones du muscle à stimuler. On pourra notamment se reporter aux brevets EP938910 (MO), EP733381 (MAUER), FR2767481 (MAMBERTI), FR2754717 (CARPRIEAUX), FR2712498 (GELEC), WO9903421 (UTAH), FR2673112 (PEZ) et US5456709 (HAMEDI) qui décrivent de telles sondes.

Toutefois, le positionnement et l'immobilisation de ces sondes d'un deuxième type à l'intérieur de la cavité du patient ne sont pas satisfaisants. En effet, les muqueuses du muscle à stimuler sont humides, ce qui favorise en cela un déplacement de la sonde de conformation globale cylindrique. Cette immobilisation et ce positionnement sont rendus d'autant plus délicats que la sonde est habituellement légère pour sa maniabilité, et est prévue pour être utilisée par le patient qui se trouve en différentes postures, debout, assis ou couché. Cet inconvénient est préjudiciable tant pour une localisation correcte des stimulations appliquées, que pour la fiabilité et la pertinence des informations transmises le cas échéant au centre de calcul.

Un autre problème à résoudre pour ce deuxième type de sonde réside dans l'application idoine des électrodes contre la paroi interne de la cavité. D'une manière générale, cette application est obtenue par une réaction du muscle vaginal ou du sphinctère anal à l'introduction de la sonde, qu'il tend à enserrer.

C'est pourquoi les concepteurs de l'art antérieur ont proposé des sondes, dont le corps est élastiquement déformable, à l'encontre de la contraction du muscle qui tend à l'enserrer, de manière à plaquer les électrodes contre la paroi intérieure du muscle et à immobiliser la sonde. On pourra notamment se reporter aux brevets US5370671 (EMPI) et WO9736643 (IOTEK), qui décrivent des sondes du genre. GB-A-2 284 991 décrit une sonde comprenant un corps allongé et élastiquement déformable qui, pour résister aux efforts auxquels il est soumis, doit être réalisé dans un matériau relativement rigide, au point qu'il peut blesser un patient. En outre, le bord arrière du corps de la sonde ne s'oppose pas efficacement à une introduction totale de celle-ci dans la cavité, d'où un risque de gêne ou de blessure pour le patient.

Le but de la présente invention est de proposer une sonde endocavitaire pour électrothérapie, et plus particulièrement pour la rééducation périnéo-sphinctérienne, qui offre des solutions satisfaisantes aux problèmes susvisés.

La sonde endocavitaire pour électrothérapie de la présente invention comprend un corps globalement allongé qui supporte localement à sa périphérie au moins une électrode. Ce corps supporte en outre des moyens de raccordement de cette électrode à une source de tension, et/ou accessoirement à un centre de calcul.

Le corps est élastiquement déformable, de manière à offrir une résistance élastique permanente à l'encontre des efforts produits par la cavité en réaction à l'introduction de la sonde. On comprendra que cette élasticité est telle que le corps est maintenu sous tension quelle que soit la conformation de la cavité à un quelconque instant, en raison de la faculté conférée au corps à se déformer en permanence en réaction à son enserrement par le ou les muscles de la cavité.

Le corps de la sonde de l'invention est plus particulièrement principalement structuré à partir d'un élément filiforme, qui est recourbé sur lui-même de manière à articuler l'un à l'autre ses deux brins d'extrémité. On comprendra que ces dispositions sont telles que l'élasticité du corps résulte d'un rapprochement l'un vers l'autre sous tension des brins d'extrémité de l'élément filiforme à son extrémité distale, sous l'effet de l'enserrement du corps par le muscle de la cavité.

L'élément filiforme est au moins partiellement noyé de l'intérieur d'une masse de matière souple, ce qui limite les risques de blessure. En outre, cette masse de matière souple se prolonge, à son extrémité distale, sous la forme d'un organe de mise en butée extérieure du corps de la sonde contre le patient, ce qui limite l'enfoncement de la sonde dans la cavité, le caractère souple de la matière assurant un confort d'utilisation satisfaisant.

Il résulte de ces dispositions que la sonde introduite à l'intérieur de la cavité est immobilisée. Il en résulte finalement à la fois une immobilisation spontanée de la sonde à l'intérieur de la cavité, en une position idoine, et un plaquage permanent des électrodes contre sa paroi interne.

Il en découle une exploitation maximale et certaine des effets produits par les électrodes, localisées en une position déterminée sur le corps, et le cas échéant une pertinence et une fiabilité des informations transmises au centre de calcul.

Selon diverses variantes, soit l'élément filiforme est monobloc, son élasticité résultant de la zone de courbure entre ses brins d'extrémité, soit l'élément filiforme est composé des deux brins d'extrémité assemblés l'un à l'autre par un organe d'articulation, tel qu'une masse souple.

Selon une forme perfectionnée de réalisation, les deux brins de l'élément filiforme sont assemblés l'un à l'autre par l'intermédiaire d'un organe d'articulation équipé de moyens de réglage de leur débattement l'un par rapport à l'autre. Ces moyens de réglage visent à permettre d'ajuster l'écart initial entre les deux brins d'extrémité selon la taille de la cavité, et/ou selon la position de l'utilisateur utilisant la sonde. Il en découle que selon le réglage initial de cet écart, et le positionnement de la sonde à l'intérieur de la cavité, divers muscles de cette cavité peuvent être sollicités, selon le traitement spécifiquement recherché.

La section des brins d'extrémité de l'élément filiforme est par exemple sensiblement circulaire, plus ou moins aplatie. On notera que cette section peut être variable suivant la longueur de l'élément filiforme.

L'élément filiforme est notamment équipé à son extrémité distale d'au moins un organe de butée à l'intérieur de la cavité pour éviter un rejet de la sonde.

Selon une forme avantageuse de réalisation, l'élément filiforme est au moins partiellement noyé, ou de manière analogue manchonné ou équivalent, à l'intérieur d'une masse de matière souple en élastomère notamment ou analogue. L'électrode au moins affleure à la périphérie de cette masse souple. On comprendra que cette masse souple peut, selon diverses variantes, être homogène ou être formée à partir de matières différentes, afin notamment d'offrir des zones plus souples que d'autres.

On notera que l'électrode peut être, selon diverses variantes prises seules ou en combinaison, soit du type de conformation annulaire, en entourant le corps de la sonde, soit du type conformée en barrette, en étant localement disposée suivant la longueur du corps.

On notera aussi que les électrodes sont préférentiellement au moins nombre de deux, par exemple disposées au voisinage l'une de l'autre ou respectivement sur l'un et l'autre des brins d'extrémité.

Selon une variante associant ces deux dispositions, les électrodes sont au nombre de quatre, réparties par couple respectif sur chacun des brins d'extrémité.

On comprendra d'une manière générale, que le nombre et la disposition sur le corps des électrodes est déterminé suivant les résultats à atteindre par le traitement et corrélativement la position souhaitée de ces électrodes contre le muscle de la cavité.

La masse de matière souple se prolonge avantageusement au-delà de l'extrémité distale de l'élément filiforme, pour former l'organe de mise en butée extérieure du corps contre le patient.

De préférence, la liaison entre l'électrode et le corps est elle-même élastique, pour permettre une mobilité relative de l'électrode. On notera que le cas échéant, la masse de matière souple qui est interposée entre l'élément filiforme et l'électrode au moins, est avantageusement mise à profit, par sa souplesse, pour obtenir cette mobilité relative de l'électrode.

La présente invention sera mieux comprise et des détails en relevant apparaîtront, à la description qui va en être faite en relation avec les figures annexées, dans lesquelles :

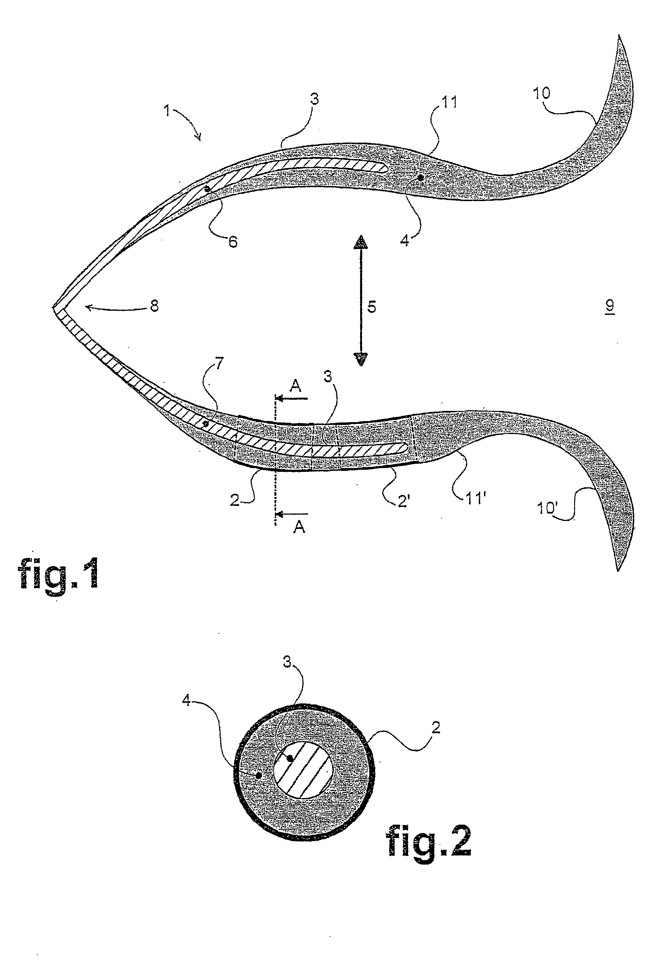

La fig.1 est une coupe longitudinale d'une sonde selon un premier mode de réalisation de l'invention,
La fig.2 est une section à plus grande échelle selon la ligne A-A de l'un des brins d'extrémité du corps de la sonde illustrée sur la fig.1,
La fig. 3 est une vue de côté, avec coupe partielle, pour une sonde conforme à un second mode de réalisation de l'invention,
La fig. 4 est une section à plus grande échelle selon la ligne B-B à la figure 3 et
La fig. 5 est une section à plus grande échelle selon la ligne C-C à la figure 3 .

Sur les figures, une sonde endocavitaire pour électrothérapie comprend un corps 1 globalement allongé supportant un couple d'électrodes 2,2'. Ce corps 1 est principalement composé d'un élément filiforme rigide 3 qui est recourbé sur lui même, et qui est noyé à l'intérieur d'une masse souple en élastomère 4.

Les électrodes 2 et 2' sont reliées par des fils non représentés à une source de tension également non représentée, ce qui leur permet d'appliquer une tension prédéterminée sur les muscles d'une cavité dans laquelle est introduite la sonde. Les électrodes peuvent également être reliées à un centre de calcul, ce qui permet de mesurer la tonicité des muscles.

La structure du corps 1 formé à partir de l'élément filiforme 3, lui confère une élasticité visant au rapprochement ou à l'éloignement spontané (flèche 5) de ses brins d'extrémité 6 et 7, sous l'effort produit par les muscles de la cavité en réaction à l'introduction de la sonde.

On remarquera la zone 8 d'articulation entre les deux brins d'extrémité 6 et 7 de l'élément filiforme 3, située à l'extrémité proximale de la sonde. On comprendra que la sonde est introduite par cette extrémité proximale à l'intérieur de la cavité du patient.

La masse souple 4 enveloppant l'élément filiforme prolonge ses brins d'extrémité 6 et 7 vers l'extrémité distale 9 de la sonde. Ce prolongement est agencé à son extrémité distale en organe 10,10' d'appui axial de la sonde contre le patient, à l'extérieur de la cavité. Par ailleurs, cette masse souple 4 comporte des renflements 11 et 11' au voisinage de son extrémité distale 9, qui constituent des organes de butée interne de la sonde à l'intérieur de la cavité du patient, pour éviter son rejet spontané.

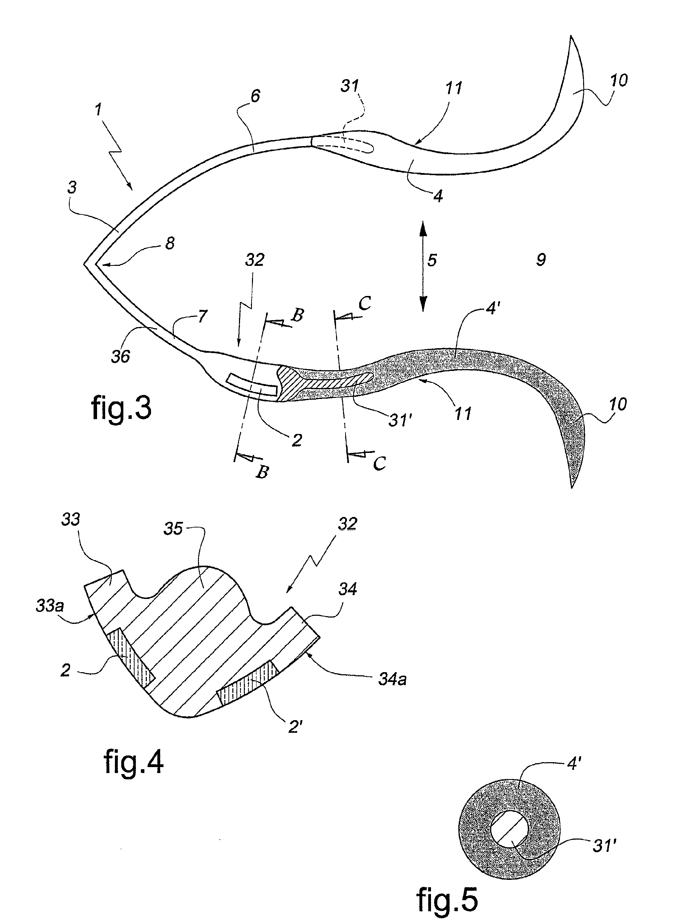

Dans le second mode de réalisation de l'invention représenté aux figures 3 à 5, les éléments analogues à ceux du premier mode de réalisation portent des références identiques.

Le corps 1 de ce mode de réalisation est globalement allongé et forme une zone 8 d'articulation entre ses deux brins 6 et 7, ce qui permet un rapprochement et un éloignement de ceux-ci, comme représenté par la double flèche 5.

Le corps 1 comprend un élément filiforme 3 réalisé dans un matériau suffisamment rigide ou semi-rigide pour résister efficacement aux efforts de rapprochement des brins 6 et 7. L'élément 3 peut, par exemple, être réalisé en ABS ou en résine acétale du genre DELRIN (marque commerciale).

Au niveau de chacune de ses extrémités 31 et 31', l'élément 3 est noyé dans une masse 4 ou 4' de matière souple, telle qu'un élastomère, ce qui améliore le confort d'utilisation de la sonde. Les masses souples 4 et 4' prolongent donc les brins 6 et 7 vers l'extrémité distale 9 de la sonde et constituent des organes 10 et 10' d'appui axial de la sonde contre le patient, à l'extérieur de la cavité, ce qui permet de limiter l'introduction de la sonde dans la cavité. Par ailleurs, les masses souples 4 et 4' comportent des renflements 11 et 11' ménagés sur leurs parties externes, au voisinage de l'extrémité 9, et qui s'opposent à une éjection de la sonde par rapport à la cavité.

Comme il ressort plus particulièrement de la figure 4, l'élément 3 a, dans une zone 32 où il est équipé de deux électrodes 2 et 2', une forme globalement en V, avec deux branches 33 et 34 s'étendant de part et d'autre d'une âme centrale 35 prolongeant une partie globalement circulaire 36 constituant l'essentiel de la longueur du brin 7. Les électrodes 2 et 2' sont disposées de façon à être affleurantes par rapport aux surfaces externes 33a et 34a des branches 33 et 34.

On comprend que, dans ce cas, les masses des matières souples 4 et 4' n'entourent pas l'élément filiforme 3 au niveau des électrodes 2 et 2'.

Quel que soit le mode de réalisation considéré, les électrodes 2 et 2' peuvent être réalisées sous la forme d'éléments tubulaires, par exemple en acier inoxydable, à l'intérieur desquels sont moulés la masse 4 dans le premier mode de réalisation ou l'élément 3 dans le second mode de réalisation. Ces éléments tubulaires ont une épaisseur de l'ordre de 1 à 2 mm.

En variante, les électrodes 2 et 2' peuvent être formées par un dépôt électrolytique, par exemple à base d'or, avec une épaisseur de quelques µm, ce qui permet d'obtenir une très faible résistivité pour ces électrodes. Cette faible résistivité améliore les capacités de mesure d'une sonde conforme à l'invention reliée à un centre de calcul et les possibilités de stimulation d'une sonde reliée à une source de tension. En particulier, les picotements éventuellement ressentis par le patient sont sensiblement diminués par rapport au cas où l'on utilise des électrodes tubulaires.

Les caractéristiques techniques des différents modes de réalisation représentés peuvent être combinées entre elles dans le cadre de la présente invention.

## Revendications

1. Sonde endocavitaire pour électrothérapie, du genre de sonde comprenant un corps (1) globalement allongé et élastiquement déformable (5) de manière à offrir une résistance élastique permanente à l'encontre des efforts produits par la cavité en réaction à l'introduction de la sonde, ce corps (1) étant principalement structuré à partir d'un élément filiforme (3) qui est recourbé sur lui-même, de manière à articuler l'un à l'autre ses deux brins d'extrémité (6,7), l'élasticité du corps (1) résultant d'un rapprochement l'un vers l'autre sous tension des brins d'extrémité (6,7) de l'élément filiforme (3) à son extrémité distale, ce corps supportant localement à sa périphérie au moins une électrode (2,2'), ladite sonde comprenant également des moyens de raccordement de cette électrode à une source de tension et/ou à un centre de calcul, **caractérisée en ce que** l'élément filiforme (3) est au moins partiellement noyé dans l'intérieur d'une masse de matière souple (4) qui se prolonge, à son extrémité distale (9), sous la forme d'un organe (10, 10') de mise en butée extérieure du corps (1) de la sonde contre le patient.

2. Sonde endocavitaire selon la revendication 1, **caractérisée :**
**en ce que** l'élément filiforme (3) est monobloc, son élasticité résultant de la zone de courbure entre ses brins d'extrémité (6,7).

3. Sonde endocavitaire selon la revendication 1, **caractérisée :**
**en ce que** l'élément filiforme (3) est composé des deux brins d'extrémité assemblés l'un à l'autre par un organe d'articulation.

4. Sonde endocavitaire selon la revendication 3, **caractérisée :**
**en ce que** l'organe d'articulation est équipé de moyens de réglage du débattement des brins d'extrémité de l'élément filiforme l'un par rapport à l'autre.

5. Sonde endocavitaire selon l'une quelconque des revendications précédentes, **caractérisée :**
**en ce que** la section des brins d'extrémité (6,7) de l'élément filiforme (3) est sensiblement circulaire.

6. Sonde endocavitaire selon l'une quelconque des revendications précédentes, **caractérisée :**
**en ce que** la section de l'élément filiforme (3) varie suivant sa longueur.

7. Sonde endocavitaire selon l'une quelconque des revendications précédentes, **caractérisée :**
**en ce que** l'élément filiforme (3) est équipé à son extrémité distale d'au moins un organe de butée (11,11') à l'intérieur de la cavité pour éviter un rejet de la sonde.

8. Sonde endocavitaire selon l'une quelconque des revendications précédentes, **caractérisée :**
**en ce que** ladite électrode (2, 2') affleure à la périphérie de ladite masse de matière souple.

9. Sonde endocavitaire selon l'une des revendications précédentes, **caractérisée :**
**en ce que** la masse de matière souple (4) se prolonge au-delà de l'extrémité distale de l'élément filiforme (3) pour former ledit organe (10,10') de mise en butée.

10. Sonde endocavitaire selon l'une quelconque des revendications précédentes, **caractérisée :**
**en ce que** la liaison entre l'électrode au moins (2,2') et le corps (1) est elle-même élastique, pour permettre une mobilité relative de l'électrode (2,2').
